# EUROPEAN PATENT APPLICATION

(11) **EP 2 462 868 A1**
(43) Date of publication of application: **13.06.2012**
(21) Application number: 10193915.5
(22) Date of filing: 07.12.2010
(51) Int. Cl.: A61B 5/055, A61B 6/04, A61G 13/06, B66F 7/06

(54) **Scissor mechanism, subject table, and medical imaging system**

(71) Applicant: Koninklijke Philips Electronics N.V., 5621 BA Eindhoven (NL)
(72) Inventor: Lenting, Gert-Jan, 5600 AE, Eindhoven (NL)
(74) Representative: Damen, Daniel Martijn

(57) **Abstract**

A subject table (600) comprising: a subject support (602) for receiving a subject (702) and a scissor mechanism (100, 200) for raising and lowering the subject support. The scissor mechanism comprises a first scissor link (104) and a second scissor link (102). The scissor mechanism further comprises a scissor pivot (106) which joins the first scissor link and the second scissor link. The second scissor link comprises a drive member (134). The scissor mechanism further comprises a linear drive element (138). The scissor mechanism is adapted such that linear motion of the linear drive element causes the drive member to follow a predetermined path (134).

## Description

### TECHNICAL FIELD

The invention relates to a scissor mechanism, in particular a scissor mechanism for raising and lowering a subject support.

### BACKGROUND OF THE INVENTION

Subject tables may be used for positioning subjects in medical imaging system. In building patient tables for various imaging modalities (e.g. MR/CT/NM/RT), a scissor mechanism is often used to control the height of a subject support. Scissor mechanisms offer a compact design in its lowest position and a large stroke. The large stroke calls for the use of a possibly telescopic actuator or an actuator acting on part of the scissor giving it a non constant transmission ratio between drive and motion. The non constant ratio requires a higher driving force on one end of the stroke than on the other.

### SUMMARY OF THE INVENTION

The invention provides for a subject table, a medical imaging system, and a scissor mechanism in the independent claims. Embodiments are given in the dependent claims.

Embodiments of the invention may solve the above mentioned problem and others by using a linear drive element to actuate the scissor mechanism. Embodiments of such a scissor mechanism may have the advantage that the transmission ratio between drive and motion at any position in its stroke may be freely defined. Additionally the use of such a drive element may also have the advantage of being able to actuate a scissor mechanism in a compact manner. The tailoring of the transmission ratio may also allow control of the stiffnes of the scissor/drive combination. Embodiments of the invention may provide for a scissor mechanism with an improved stiffness compared with known scissor mechanisms.

The linear drive element may also be referred to as a cross carriage. Some embodiments may have a cross carriage moving in horizontal direction to drive a drive member or wheel on the scissor mechanism.

'Medical image data' as used herein encompasses two or three dimensional data that has been acquired using a medical imaging system. A medical imaging system as used herein encompasses an apparatus adapted for acquiring information about the physical structure of a patient and construct sets of two dimensional or three dimensional medical image data. Medical image data can be used to construct visualizations which are useful for diagnosis by a physician. This visualization can be performed using a computer.

Magnetic Resonance Image (MRI) data is defined herein as being the recorded measurements of radio frequency signals emitted by atomic spins by the antenna of a Magnetic resonance apparatus during a magnetic resonance imaging scan. A Magnetic Resonance Imaging (MRI) image is defined herein as being the reconstructed two or three dimensional visualization of anatomic data contained within the magnetic resonance imaging data. This visualization can be performed using a computer.

A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network.

'Computer memory' or 'memory' is an example of a computer-readable storage medium. Computer memory is any memory which is directly accessible to a processor. Examples of computer memory include, but are not limited to: RAM memory, registers, and register files.

'Computer storage' or 'storage' is an example of a computer-readable storage medium. Computer storage is any non-volatile computer-readable storage medium. Examples of computer storage include, but are not limited to: a hard disk drive, a USB thumb drive, a floppy drive, a smart card, a DVD, a CD-ROM, and a solid state hard drive. In some embodiments computer storage may also be computer memory or vice versa.

A 'computing device' as used herein encompasses to any device comprising a processor. A 'processor' as used herein encompasses an electronic component which is able to execute a program or machine executable instruction. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. Many programs have their instructions performed by multiple processors that may be within the same computing device or which may even be distributed across multiple computing devices.

A 'user interface' as used herein encompasses an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, and accelerometer are all examples of user interface components which enable the receiving of information or data from an operator.

A 'hardware interface' as used herein encompasses a interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, parallel port, IEEE 1284, serial port, RS-232 port, IEEE-488 port, Bluetooth connection, Wireless local area network connection, TCP/IP connection, Ethernet connection, control voltage interface, MIDI interface, analog input interface, and digital input interface.

In one aspect the invention provides for a subject table for a medical imaging system. The subject table comprises a subject support for receiving a subject. The subject table further comprises a scissor mechanism for raising and lowering the subject support. The scissor mechanism comprises a first scissor link and a second scissor link. The scissor mechanism further comprises a scissor pivot which joins the first scissor link and the second scissor link. A pivot as used herein encompasses a joint between two or more links or elements which allows rotational motion between the joint links or elements. The second scissor link comprises a drive member. A drive member as used herein encompasses a protrusion or extension of a link which may be used for driving the motion of said link. In some instances the drive member may be a rigid extension of the second scissor link. In other embodiments the drive member may be a wheel or bearing that is mounted to a protrusion or is mounted directly to the second scissor link.

The scissor mechanism further comprises a linear drive element. A linear drive element as used herein encompasses a mass or mechanism element which is actuated or moved in a linear fashion. The scissor mechanism is adapted such that the linear motion of the linear drive element causes the drive member to follow a predetermined path. An equivalent way of stating this is that the linear drive element drives or actuates the drive member. Such a mechanism has the advantage that a linear motion of the drive element may be converted into motion which causes the scissor mechanism to raise or lower the subject support. This may be particularly advantageous when there is minimal space to implement or provide for a mechanism for raising or lowering the subject support.

In order to maximize the stroke of the cross carriage vs. the stroke of the scissor mechanism (i.e. to minimize the required force to move the cross carriage as function of the load on the scissor), a drive member may be positioned. The drive member may be positioned such that the scissor mechanism satisfies the following conditions at the same time:
- In any vertical position of the scissor, including lowest and highest position, the drive member remains within the space available for the total construction in lowest position.
- The horizontal displacement of the drive member between lowest and highest position of the scissor shall be as large as possible.

To minimize the force on the drive member (maximize stiffness, minimize material usage per same strength), it is preferred to also comply with following condition:
- The vertical displacement of the drive member between lowest and highest position of the scissor shall be as large as possible. In this synthesis it is assumed that the cross carriage moves in the plane between the lower scissor joints. The cross carriage can also move in the plane between the upper scissor joints, in that case the mechanism can be inverted after synthesis of the construction.

In some embodiments, the drive member is placed such that there is a constant ratio between vertical and horizontal forces and displacement. This constant ratio between horizontal and vertical displacement and/or forces is also possible in cases where the ratio between lowest and highest position of the scissor is much greater than 2.

The transmission ratio between the height movement of a scissor mechanism and the drive may be tailored in some embodiments for instance: The construction can be extended with a non constant transmission ratio to improve the stiffness at the end or high position of the scissor. The essence of the invention is that a linear drive element can be added to a scissor mechanism to provide the freedom to set a freely defined transmission ratio between drive and motion at any position in its stroke.

In another embodiment the scissor mechanism further comprises a first parallel link and a second parallel link. The scissor mechanism further comprises a first sliding element and a second sliding element. The scissor mechanism further comprises a first parallel pivot for joining the first parallel link with the first scissor link. The scissor mechanism further comprises a second parallel pivot for joining the second parallel link and the second scissor link. The scissor mechanism further comprises a first sliding pivot for joining the first sliding element and the second scissor link. The scissor mechanism further comprises a second sliding pivot for joining the second sliding link.

In another embodiment a first axis through the first parallel pivot and the first sliding pivot remains parallel to a second axis through the second parallel pivot and the second sliding pivot. This embodiment places a restraint on the mechanism. The advantage of such a limitation is that the scissor mechanism will raise and lower the subject support in a manner such that the subject support is always raised without tilting the subject support.

In another embodiment the distance between the first parallel pivot and the second sliding pivot is identical to the distance between the first sliding pivot and the second parallel pivot.

In another embodiment the linear drive element is adapted such that there is a constant transmission ratio between motion of the linear drive element and motion of the scissor mechanism. In other words the ratio of the distance that the linear drive element moves to the distance that the scissor mechanism moves or lifts, perpendicular to the motion of the linear drive element, is a constant ratio. This has the advantage that a constant force is able to actuate the scissor mechanism.

In another embodiment the linear drive element is adapted such that the transmission ratio between motion of the linear drive element and motion of the scissor mechanism varies over the stroke of the linear drive element. For instance there may be regions of the stroke which have distinct transmission ratios.

In another embodiment the scissor pivot is located at the mid-point between the first sliding pivot and the second scissor link.

In another embodiment the scissor pivot is located at the mid-point between the second sliding pivot and the first parallel pivot.

In another embodiment the linear drive element comprises a sloped surface. The sloped surface is sloped relative to the direction of linear motion of the linear drive element. The sloped surface causes the drive member to follow the predetermined path. Essentially as the near drive element is moved in a linear fashion the sloped surface comes in contact with the drive member and drives it along the predetermined path.

In another embodiment the drive member is constrained to the sloped surface by a restoring force. This may be realized in several different ways. For instance the gravitational force may be used as the restoring force. In other embodiments means such as a spring may be used. For instance a spring between the second scissor link and the second parallel link and/or a spring between the first scissor link and the second parallel link may be used to provide the restoring force.

In another embodiment the driving member comprises a track. A track as used herein encompasses a recess or a groove which is used to both guide and drive the drive member along the predetermined path. The track is adapted for receiving the drive member and for driving the drive member along the predetermined path.

In another embodiment the subject table further comprises a linear actuator for moving the linear drive element. The linear actuator may be realized in many different ways. For instance a pneumatic or hydraulic system may be used for providing the linear motion. In other embodiments the linear actuator may be the combination of a rotational motor and a ball screw.

In another embodiment the linear actuator is a rotational motor. The rotational motor is connected to a clutch. The clutch is adapted for selectively rotating a horizontal motion driveshaft and a vertical motion driveshaft. The vertical motion driveshaft is adapted for causing the linear motion of the linear drive element. The horizontal driveshaft is adapted for driving the subject support in a horizontal direction. This embodiment is particularly advantageous because a single rotational motor is able to provide both horizontal and vertical motion of the subject support.

In another aspect the invention provides for a medical imaging system comprising a subject table according to an embodiment of the invention. A medical imaging system as used herein is a system for acquiring medical image data.

In another embodiment the medical imaging system is adapted for acquiring medical image data from an imaging zone. The subject table comprises a linear actuator for moving the linear drive element. The linear actuator is a rotational motor. The rotational motor is connected to a clutch. The clutch is adapted for selectively rotating a horizontal motion driveshaft and a vertical motion driveshaft. The vertical motion driveshaft is adapted for causing the linear motion of a linear drive element. The horizontal driveshaft is adapted for driving the subject support in a horizontal direction.

The medical imaging system further comprises a subject table controller. The subject table controller is adapted for controller the rotational motor and clutch such that the rotational motion of the vertical motion driveshaft causes vertical motion of the subject support relative to the imaging zone. The subject table controller is adapted for controlling the rotational motor and clutch such that the rotational motion of the horizontal motion driveshaft causes horizontal motion of the subject support relative to the imaging zone.

In another embodiment the medical imaging system is a magnetic resonance imaging system.

In another embodiment the medical imaging system is a positron emission tomography system.

In another embodiment the medical imaging system is a computed tomography system.

In another aspect the invention provides for a scissor mechanism comprising a first scissor link and a second scissor link. The scissor mechanism further comprises a scissor pivot which joins the first scissor link and the second scissor link. The first scissor link comprises a drive member. The scissor mechanism further comprises a linear drive element. The scissor mechanism is adapted such that the linear motion of the linear drive element causes the drive member to follow a predetermined path.

In another embodiment the scissor mechanism comprises a first parallel link element and a second parallel link element. The scissor mechanism further comprises a first sliding element and a second sliding element. The scissor mechanism further comprises a first parallel pivot for joining the first parallel link and the first scissor link. The second scissor mechanism further comprises a second parallel pivot for joining the second parallel link and the second scissor link. The scissor mechanism further comprises a first sliding pivot for joining the first sliding element and the second scissor link. The scissor mechanism further comprises a second sliding pivot for joining the second sliding link.

In another embodiment a first axis through the first parallel pivot and the first sliding pivot remains parallel to a second axis through the second parallel pivot and the second sliding pivot.

### BRIEF DESCRIPTION OF THE DRAWINGS

In the following preferred embodiments of the invention will be described, by way of example only, and with reference to the drawings in which:
Fig. 1 illustrates a scissor mechanism according to an embodiment of the invention;
Fig. 2 illustrates a scissor mechanism according to a further embodiment of the invention;
Fig. 3 illustrates a scissor mechanism according to a further embodiment of the invention;
Fig. 4 illustrates a scissor mechanism according to a further embodiment of the invention;
Fig. 5A through 5D illustrates a scissor mechanism according to a further embodiment of the invention;
Fig. 6 illustrates a subject table according to an embodiment of the invention;
Fig. 7 illustrates a medical imaging system according to an embodiment of the invention; and
Fig. 8 illustrates a magnetic resonance imaging system according to an embodiment of the invention.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

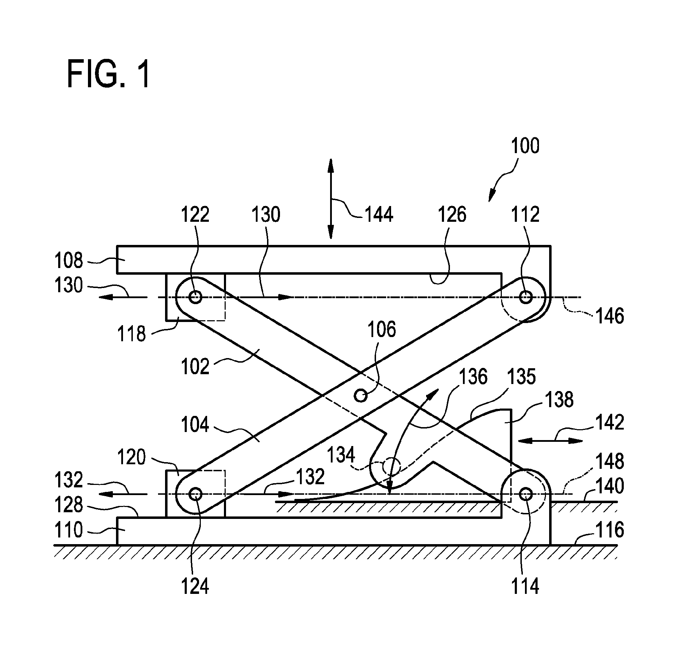

Fig. 1 shows an embodiment of a scissor mechanism 100 according to the invention. Shown in Fig. 1 there is a first scissor link 104 and a second scissor link 102. The first scissor link 104 and the second scissor link 102 are joined by a scissor pivot 106. Also shown is a first parallel link 108 and a second parallel link 110. There is a first parallel pivot 112 which joins the first parallel link 108 with the first scissor link 104. There is a second parallel pivot 114 which joins the second scissor link 102 with the second parallel link 110.

The second parallel link 110 is shown as resting on a rigid support 116. In some embodiments the first parallel link 108 may instead be resting on a rigid support. In still yet other embodiments neither the first parallel link 108 nor the second parallel link 110 is resting on a rigid support. Shown in the Fig. is a first sliding element 118 and a second sliding element 120. There is a first sliding pivot 122 which joins the first sliding element 118 to the second scissor link 102. There is a second sliding pivot 124 that joins the second sliding element 120 with the first scissor link 104. In this Fig. the first sliding element 118 and the second sliding element 120 are representative of elements or components that slide along or are guided by the parallel links 108, 110.

In this Fig. the first sliding element 118 is constrained to moving along the surface 126 of the first parallel link 108. The second sliding element 120 is constrained to moving along the surface 128 of the second parallel element 110. This representation is intended to be purely representational. It is understood that such a joint may be implemented in a variety of ways. There may be for instance a track or a groove in one or both of the parallel links 108, 110. The sliding elements 118, 120 may be preferably replaced with a wheel or a roller bearing. The arrows 130 show the motion of the first sliding element 118. The arrows 132 show the motion of the second sliding element 120. Shown in the Fig. is a drive member 134 on the second scissor link 102. The drive member 134 may for instance be a protrusion or it may be a wheel or roller bearing mounted to the second scissor link 102.

The entire second scissor link 102 rotates about the second parallel pivot 114. Therefore the drive member 134 follows a circular path 136 about the second parallel pivot 114. This may be considered to be a predetermined path that the drive member 134 follows. There is a linear drive element 138 with a sloped surface 135 in contact with the drive member 134. The linear drive element 138 is constrained to move on a second rigid surface 140. In some embodiments, the second rigid surface 140 may be identical with the rigid support 116. In other embodiments the second rigid surface may be one of the parallel links 108, 110. The constraint of the linear drive element 138 to the second rigid surface 140 is intended to represent linear motion 142 of the linear drive element 138. The arrows 142 show the direction of the linear motion of the linear drive element. A sloped surface 135 of the linear drive element 138 is in contact with the drive member 134. As the linear drive element 138 is driven in a linear fashion the drive member 134 is driven along the path 136. Motion of the drive member 134 along the path 136 drives the link and causes motion of the scissor mechanism 144 in a vertical direction. It can be seen that in this embodiment the motion of the scissor mechanism 144 is perpendicular to the linear motion 142. However, it is not necessary for the linear motion along path 136 to be perpendicular to the direction 144.

In this particular embodiment the distance between the second sliding pivot and the first parallel pivot is identical to the distance between the first sliding pivot 122 and the second parallel pivot 114. Additionally the scissor pivot 106 is located at a mid-point between both the first sliding pivot 122 and the second parallel pivot 114 and also at a mid-point between the second sliding pivot 124 and the first parallel pivot 112. The way this is constructed a first axis 146 through the first sliding pivot 122 and the first parallel pivot 112 always remains parallel to a second axis 148 through the second sliding pivot 124 and the second parallel pivot 114. For this particular embodiment the scissor mechanism moves in the direction 144 without tilting the mechanism.

There are several design considerations to take into consideration when designing such a mechanism. A first consideration is the location of the drive member 134. The closer the drive member 134 is mounted to the second parallel pivot 114 the less mechanical advantage there will be. However, mounting the drive member 134 further from the second parallel pivot 114 reduces the distance in the direction 144 that the scissor mechanism can travel. The angle which the drive member 134 makes with the second parallel pivot 114 and the second axis 148 also determines the mechanical advantage.

The shape of the sloped surface 135 also affects how a motion in the direction 142 is translated into a motion in the direction 144. This can be used to tailor the amount of force needed to be applied to the linear drive element 138 which results in a motion in the direction 144. In particular this can be used such that a relatively large motion of the linear drive element 138 is needed to make a small motion in direction 144 of the mechanism. This may result in the mechanism being extremely stable in comparison to conventional scissor mechanisms. In fact the scissor mechanism can be designed such that the scissor mechanism rapidly expands to a desired height and is then only moved a small amount by a further motion of the linear drive element 138. This would make a mechanism that rises rapidly and then becomes extremely stable or rigid.

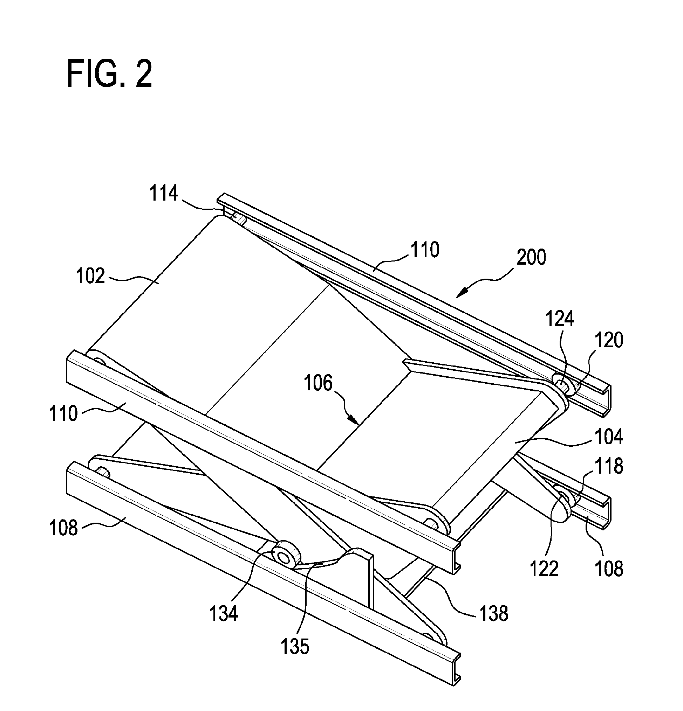

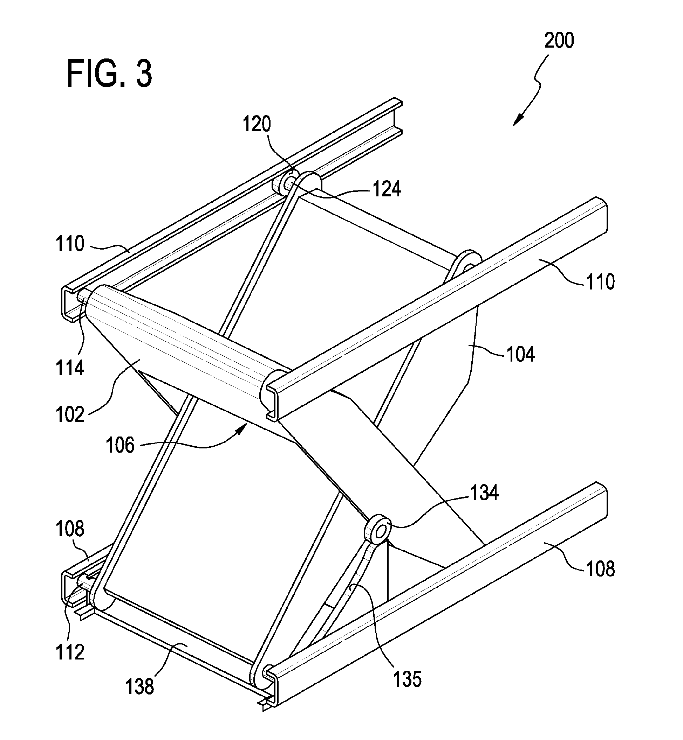

Figs. 2, 3 and 4 show renderings of a scissor mechanism 200 according to an embodiment of the invention. Elements in Figs. 2, 3 and 4 which correspond to elements in Fig. 1 are labeled using identical reference numbers. In comparing the embodiments shown in Figs. 2, 3 and 4 without a Fig. 1 there are several differences. In Fig. 1 the drive member 134 was located on the second scissor link between the scissor pivot 106 and the second parallel pivot 114. In the embodiment shown in Figs. 2, 3 and 4 the drive member 134 is located between the first sliding pivot 122 and the scissor pivot 106. Further, in the embodiments shown in Figs. 2, 3 and 4 the drive member 134 as are the first sliding element 118 and the second sliding element 122 are wheels or roller bearings. The drive member 134 is a wheel or roller bearing that travels on the sloped surface 135. The first parallel link 108 and the second parallel link 110 both form channels which the first sliding element 118 and the second sliding element 120 can travel in respectively.

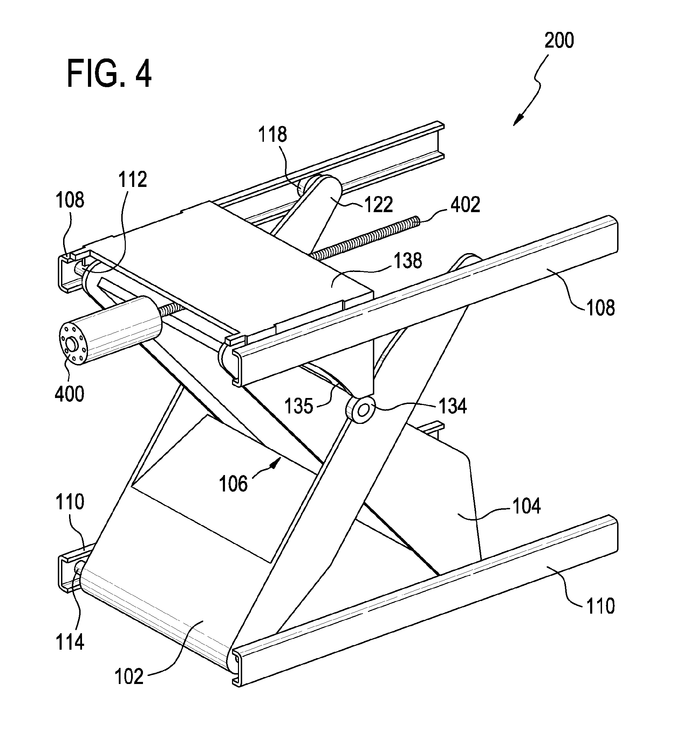

In Fig. 4 there is additionally shown a rotational motor 400 which is adapted for driving a vertical motion driveshaft 402. The vertical motion driveshaft 402 is connected to the linear drive element 138 such that when the vertical motion driveshaft 402 rotates the linear drive element 138 moves in a linear fashion. Also in the embodiments shown in Figs. 2, 3 and 4 the linear drive element 138 is shown as being movably mounted on the first parallel link 108. The linear drive element 138 can slide along the first parallel link 108.

The embodiments shown in Figs. 2, 3 and 4 the mechanism can be oriented with the drive mechanism on the top or the bottom. The configuration of the carriage drive on the top can easily be extended with a clutch drive to drive a second horizontal spindle to drive a table top into the image bore or imaging region of a medical imaging system. The carriage or driving element may be guided in the same U-channel that was used to guide the sliding elements 118, 120. This may help reduce costs of adding the carriage as an additional element. The linear drive element may be driven by a ball screw or a lead screw. The direct drive of a ball screw is also possible for example driving it at the rate of 100 mm/s at approximately 3,000 rpm. Embodiments of such a mechanism are that there is move space available in the direction that the linear drive element travels than in the direction that the scissor mechanism travels.

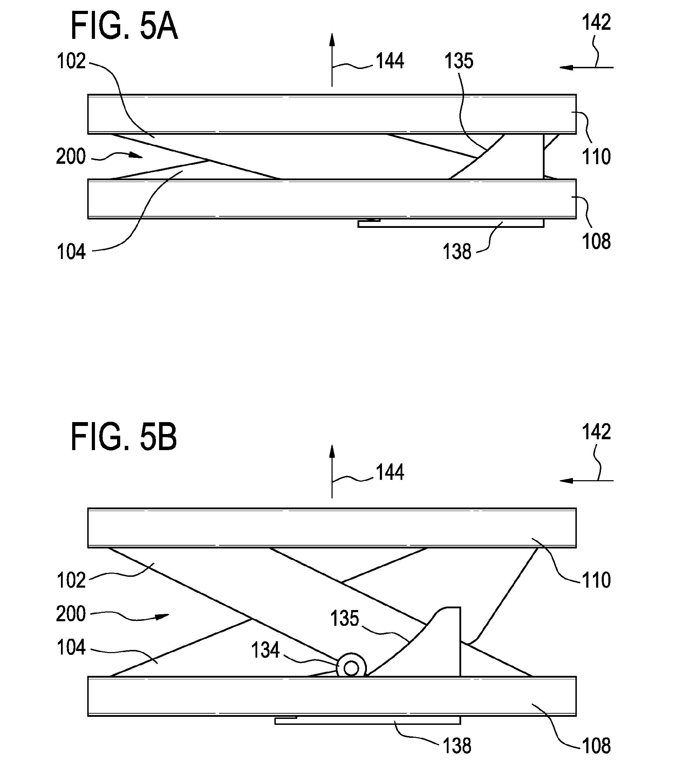

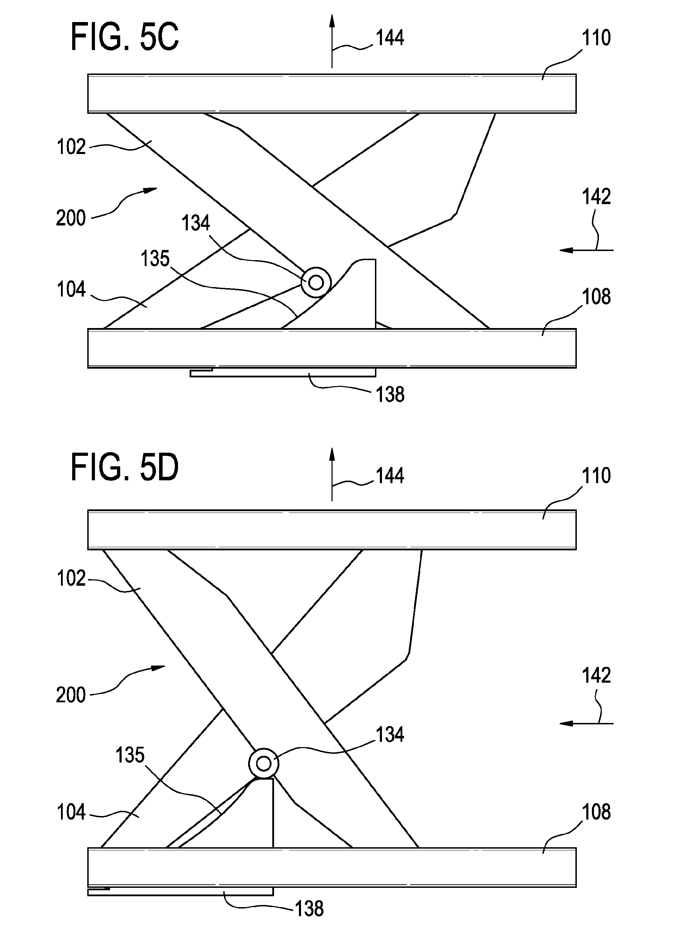

Figs. 5a, 5b, 5c and 5d show a side view of the same embodiments shown in Figs. 2-4. In the embodiment shown in Figs. 5a-5d the linear drive element 138 is moved in the direction 142. As the drive element 135 moves in the direction 142 the scissor mechanism 200 expands opening in the direction 144. What is significant about this embodiment is that the displacement in the direction 142 is equivalent to the displacement in the direction 144 over the whole stroke of the linear drive element 138. The force applied to the linear drive element 138 in the direction 142 is also equivalent to the force exerted by the scissor mechanism 200 in the direction 144.

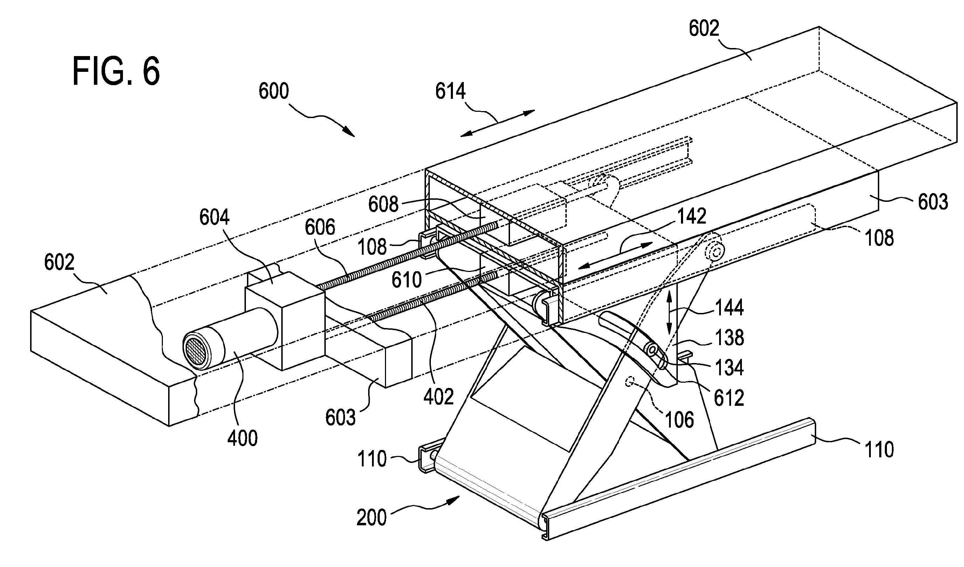

Fig. 6 shows a subject table 600 according to an embodiment of the invention. There is a subject support 602 mounted over an embodiment of a mechanism 200. There is a mechanism support 603 mounted to the mechanism 200. The subject support 602 is adapted such that it can slide horizontally in direction 614 over the mechanism support 603. There is a rotational motor 400 connected to a clutch 604. The clutch 604 is used to select between driving a vertical motion driveshaft 402 and a horizontal driveshaft 606. The clutch 604 is mounted to the mechanism support 603. The clutch 604 is under the subject support 602. The mechanism support 603 is attached to the parallel link 108.

In this embodiment a ball screw is used for both the vertical motion driveshaft 402 and the horizontal driveshaft 606. There is a ball nut 608 which is threaded along the horizontal driveshaft 606 and is connected to the subject support 602. There is another ball nut 610 which is threaded around the vertical motion driveshaft 402 and is connected to the scissor mechanism 200.

The scissor mechanism 200 is equivalent to the scissor mechanism which is illustrated in Figs. 2-5. A difference though is that there is a slot 612 in the linear drive element 138. The slot 612 serves as a guide for the drive member 134. Motion of the ball screw 608 causes motion of the subject support 602 in the direction of arrow 614. Motion of ball screw 610 causes motion of the linear drive element 138. Motion of the linear drive element 138 causes motion of the scissor mechanism in the direction 144 which effectively lifts the subj ect support 602 in a vertical direction.

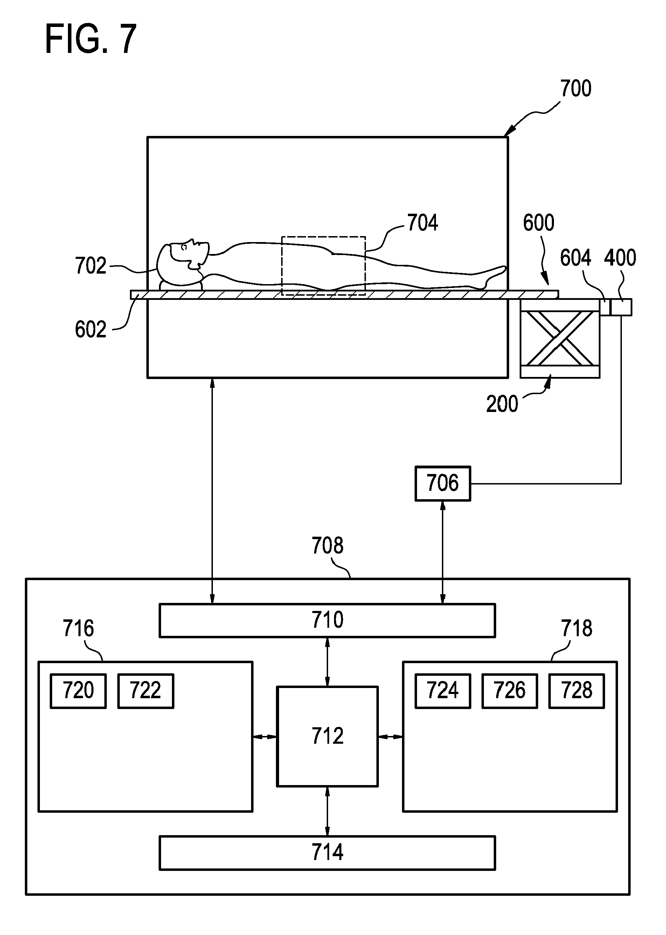

Fig. 7 shows an embodiment of a medical imaging system 700 according to an embodiment of the invention. There is a subject table 600 which comprises a scissor mechanism 200 and a subject support 602. There is a rotational motor 400 with a clutch 604 for controlling and moving the subject support 602 as was illustrated in Fig. 6. Resting on the subject support 602 is a subject 702. A portion of the subject 702 is within an imaging zone 704. The medical imaging system 700 in Fig. 7 is intended to be representative of a variety of types of medical imaging systems. For instance the medical imaging system 700 may be, but is not limited to: a magnetic resonance imaging system, a positron emission tomography system, and a computed tomography system.

The medical imaging system 700 and a subject table controller 706 are connected to a hardware interface 710 of a computer system 708. The subject table controller 706 is shown as being connected to the scissor mechanism 200. The computer system 708 further comprises a processor 712 for executing machine executable instructions. The processor is connected to the hardware interface 710, a user interface 714, computer storage 716 and computer memory 718. The hardware interface 710 allows the processor 712 to control the functioning and receive data from the medical imaging system 700 and also to control the subject table 600.

Within the computer storage 716 is medical image data 720 which was acquired from the imaging zone 704. Also within computer storage 716 is a medical image 722 which has been constructed from medical image data 720. The computer memory 718 is shown as containing machine executable instructions for controlling the operation of the medical imaging system 700. There is a control module 724 located in the computer memory 718. The control module 724 is computer executable instructions which contain software or instructions for controlling the operation and function of the medical imaging system. Through execution of these instructions the medical imaging system 700 acquires the medical image data 720. Also within the computer memory 718 is an image reconstruction module 726. The image reconstruction module 726 contains computer executable code which is able to reconstruct the medical image 722 from the medical image data 720. Also within the computer memory 718 is a subject table control module 728. The subject table control module 728 contains computer executable instructions which allow the processor 712 to send control signals to the subject table controller 706. The control signals allow the processor to control the motion of the subject support 602 and move it horizontally and vertically with respect to the imaging zone 704.

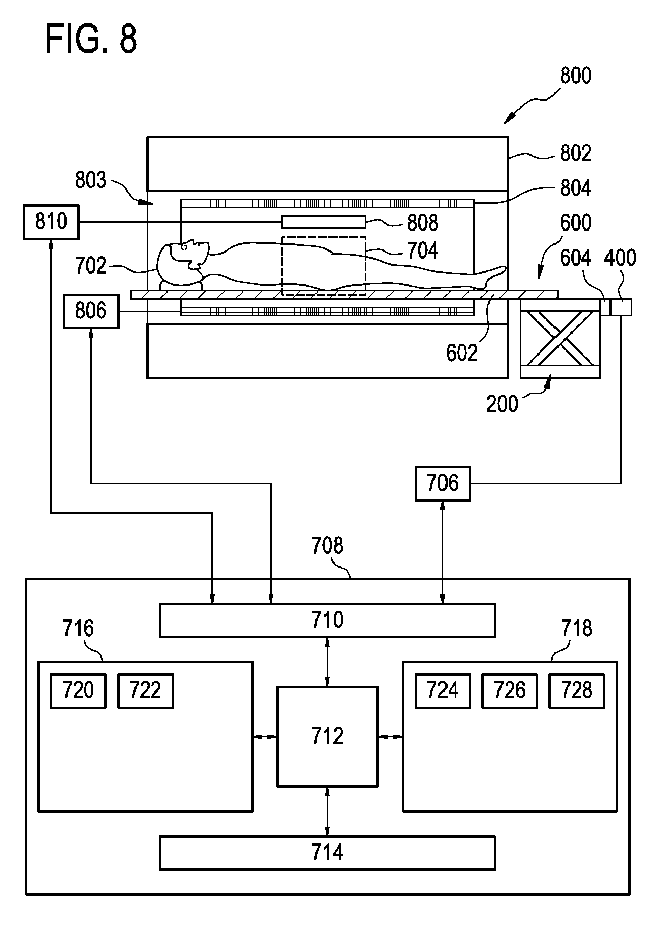

Fig. 8 shows a further embodiment of a medical imaging system according to an embodiment of the invention. In Fig. 8 the medical imaging system is a magnetic resonance imaging system 800. The magnetic resonance imaging system comprises a magnet 802. The magnet shown in Fig. 8 is a cylindrical type superconducting magnet. The magnet has a liquid helium cooled cryostat with superconducting coils. It is also possible to use permanent or resistive magnets. The use of different types of magnets is also possible for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore of the cylindrical magnet there is an imaging zone where the magnetic field is strong and uniform enough to perform magnetic resonance imaging.

The magnet 802 has a cylindrical bore 803 for receiving a subject 702. Inside of the bore 803 is a magnetic field gradient coil 804 which is used during the acquisition of magnetic resonance data to spatially encode magnetic spins within an imaging zone of the magnet. The magnetic field gradient coil 804 is connected to a magnetic field gradient coil power supply 806. The magnetic field gradient coil is intended to be representative. Typically magnetic field gradient coils contain three separate sets of coils for spatially encoding in three orthogonal spatial directions. A magnetic field gradient power supply supplies current to the magnetic field gradient coils. The current supplied to the magnetic field coils is controlled as a function of time and may be ramped or pulsed.

Within the center of the bore 803 is an imaging zone 704. Adjacent to the imaging zone 704 is a radio frequency coil 808 for manipulating the orientations of magnetic spins within the imaging zone and for receiving radio transmissions from spins also within the imaging zone. The radio frequency coil 808 may contain multiple coil elements. The radio frequency coil 808 may also be referred to as a channel or antenna. The radio frequency coil is connected to a radio frequency transceiver 810. The radio frequency coil and radio frequency transceiver may be replaced by separate transmit and receive coils and a separate transmitter and receiver. It is understood that the radio frequency coil and the radio frequency transceiver are simply representative. The radio frequency antenna is intended to also represent a dedicated transmit antenna and a dedicated receive antenna. Likewise the transceiver may also represent a separate transmitter and receivers.

As with the embodiment shown in Fig. 7 there is a subject table 600 which provides a subject support 602 for placing the subject 702. The subject 702 is located partially within the imaging zone 704. The subject table 600 is connected to a subject table controller 706. The subject table controller 706, the transceiver 810 and the magnetic field gradient coil power supply 806 are shown as being connected to a hardware interface 710 of a computer system 708. The computer system 708 and the contents of its memory 716 and storage 718 are equivalent to the computer system shown in Fig. 7. In this embodiment the medical imaging data 720 is magnetic resonance data. The medical image 722 is a magnetic resonance image.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: scissor mechanism
- 102: second scissor link
- 104: first scissor link
- 106: scissor pivot
- 108: first parallel link
- 110: second parallel link
- 112: first parallel pivot
- 114: second parallel pivot
- 116: rigid support
- 118: first sliding element
- 120: second sliding element
- 122: first sliding pivot
- 124: second sliding pivot
- 126: surface of first parallel link
- 128: surface of second parallel link
- 130: motion of first sliding element
- 132: motion of second sliding element
- 134: drive member
- 135: sloped surface
- 136: path of drive member
- 138: linear drive element
- 140: second rigid surface
- 142: motion of linear drive element
- 144: motion of scissor mechanism
- 146: first axis
- 148: second axis
- 200: scissor mechanism
- 400: rotational motor
- 402: vertical motion drive shaft
- 600: subject table
- 602: subject support
- 603: mechanism support
- 604: clutch
- 606: horizontal drive shaft
- 608: ball nut
- 610: ball nut
- 612: slot
- 614: motion of table
- 700: medical imaging system
- 702: subject
- 704: imaging zone
- 706: subject table controller
- 708: computer system
- 710: hardware interface
- 712: processor
- 714: user interface
- 716: memory
- 718: storage
- 720: medical image data
- 722: medical image
- 724: control module
- 726: image reconstruction module
- 728: subject table control module
- 800: magnetic resonance imaging system
- 802: magnet
- 803: bore of magnet
- 804: magnetic field gradient coil
- 806: magnetic field gradient coil power supply
- 808: radio frequency coil
- 810: transceiver

## Claims

1. A subject table (600) comprising:
- a subject support (602) for receiving a subject (702); and
- a scissor mechanism (100, 200) for raising and lowering the subject support, wherein the scissor mechanism comprises a first scissor link (104) and a second scissor link (102), wherein the scissor mechanism further comprises a scissor pivot (106) which joins the first scissor link and the second scissor link, wherein the second scissor link comprises a drive member (134), wherein the scissor mechanism further comprises a linear drive element (138), and wherein the scissor mechanism is adapted such that linear motion of the linear drive element causes the drive member to follow a predetermined path (134).

2. The subject table of claim 1, wherein the scissor mechanism further comprises a first parallel link (108) and a second parallel link (110), wherein the scissor mechanism further comprises a first sliding element (118) and a second sliding element (120), wherein the scissor mechanism further comprises a first parallel pivot (112) for joining the first parallel link and the first scissor link, wherein the scissor mechanism further comprises a second parallel pivot (114) for joining the second parallel link and the second scissor link, wherein the scissor mechanism further comprise a first sliding pivot (122) for joining the first sliding element and the second scissor link, wherein the scissor mechanism further comprises a second sliding pivot (124) for joining the second sliding link.

3. The subject table of claim 2, wherein a first axis (146) through the first parallel pivot and the first sliding pivot remains parallel to a second axis (148) through the second parallel pivot and the second sliding pivot.

4. The subject table of any one of the proceeding claims, wherein the linear drive element comprises a sloped surface (135), wherein the sloped surface is sloped relative to the direction of linear motion of the linear drive element, and wherein the sloped surface causes the drive member to follow the predetermined path.

5. The subject table of claim 4, wherein the drive member is constrained to the sloped surface by a restoring force.

6. The subject table of any one of claims 1 through 3, wherein the driving member comprises a track (612), and wherein the track is adapted for receiving the drive member and for driving the drive member along the predetermined path.

7. The subject table of any one of the preceding claims, subject table further comprises a linear actuator (400, 402) for moving the linear drive element.

8. The subject table of claim 7, wherein the linear actuator is a rotational motor (400), wherein the rotational motor is connected to a clutch (604), wherein the clutch is adapted for selectively rotating a horizontal motion drive shaft (606) and a vertical motion drive shaft (402), wherein the vertical motion drive shaft is adapted for causing the linear motion (142) of the linear drive element, and wherein the horizontal drive shaft is adapted for driving the subject support in a horizontal direction (614).

9. A medical imaging system (700, 800) comprising a subject table (600) according to any one of the preceding claims.

10. The medical imaging system of claim 9, wherein the medical imaging system is adapted for acquiring medical image data (720) from an imaging zone (704), wherein the subject table is according to claim 8, wherein the medical imaging system further comprises a subject table controller (706), wherein the subject table controller is adapted for controlling the rotational motor and clutch such that rotational motion of the vertical motion drive shaft causes vertical motion of the subject support relative to the imaging zone, and wherein the subject table controller is adapted for controlling the rotational motor and clutch such that rotational motion of the horizontal motion drive shaft causes horizontal motion of the subject support relative to the imaging zone.

11. The medical imaging system of claim 9 or 10, wherein the medical imaging system is any one of the following: a magnetic resonance imaging system (800), a positron emission tomography system, and a computed tomography system.

12. A scissor mechanism (100, 200) comprising a first scissor link (104) and a second scissor link (102), wherein the scissor mechanism further comprises a scissor pivot (106) which joins the first scissor link and the second scissor link, wherein the first scissor link comprises a drive member (134), wherein the scissor mechanism further comprises a linear drive element (138), and wherein the scissor mechanism is adapted such that linear motion of the linear drive element causes the drive member to follow a predetermined path.

13. The scissor mechanism of claim 12, wherein the scissor mechanism comprises a first parallel link (108) and a second parallel link (110), wherein the scissor mechanism further comprises a first sliding element (118) and a second sliding element (120), wherein the scissor mechanism further comprises a first parallel pivot (112) for joining the first parallel link and the first scissor link, wherein the scissor mechanism further comprises a second parallel pivot (114) for joining the second parallel link and the second scissor link, wherein the scissor mechanism further comprise a first sliding pivot (122) for joining the first sliding element and the second scissor link, wherein the scissor mechanism further comprises a second sliding pivot (124) for joining the second sliding link.

14. The scissor mechanism of claim 13, wherein a first axis (146) through the first parallel pivot and the first sliding pivot remains parallel to a second axis (148) through the second parallel pivot and the second sliding pivot.
